(19) Europäisches Patentamt
European Patent Office
Office européen des brevets

(11) EP 0 896 009 B1

(12) EUROPÄISCHE PATENTSCHRIFT

(45) Veröffentlichungstag und Bekanntmachung des Hinweises auf die Patenterteilung:
**09.06.2004 Patentblatt 2004/24**

(51) Int Cl.⁷: **C08G 18/02**, C08G 18/18

(21) Anmeldenummer: **98113848.0**

(22) Anmeldetag: **24.07.1998**

(54) **Verfahren zur Herstellung von Polyisocyanaten**

Process for the preparation of polyisocyanates

Procédé pour la préparation de polyisocyanates

(84) Benannte Vertragsstaaten:
**AT BE CH DE ES FR GB IT LI NL SE**

(30) Priorität: **06.08.1997 DE 19734048**

(43) Veröffentlichungstag der Anmeldung:
**10.02.1999 Patentblatt 1999/06**

(73) Patentinhaber: **Bayer MaterialScience AG**
**51368 Leverkusen (DE)**

(72) Erfinder:
- **Richter, Frank, Dr.**
**51373 Leverkusen (DE)**
- **Dieris, Carl-Gerd, Dr.**
**41539 Dormagen (DE)**
- **Mertes, Harald, Dr.**
**50670 Köln (DE)**

(56) Entgegenhaltungen:
**EP-A- 0 339 396**
**EP-A- 0 355 479**
**EP-A- 0 379 914**
**EP-A- 0 447 074**
**EP-A- 0 798 299**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 896 009 B1

Printed by Jouve, 75001 PARIS (FR)

## Beschreibung

Die Erfindung betrifft ein Verfahren zur Herstellung von Polyisocyanaten.

Die Oligo- bzw. Polymerisierung von Isocyanaten, insbesondere unter Ausbildung von NGO-Di- und Trimerstrukturen, ist seit langem bekannt. Enthalten diese Oligo- bzw. Polymeren freie NCO-Gruppen, die gegebenenfalls auch mit Blockierungsmitteln blockiert worden sein können, sind sie außerordentlich hochwertige Ausgangsstoffe für die Herstellung einer Vielzahl von Polyurethan-Kunststoffen und Beschichtungsmitteln.

Es haben sich deshalb eine Reihe technischer Verfahren zur Herstellung von Polyisocyanaten, d.h. von Oligo- bzw. Polymeren der letztgenannten Art etabliert, z.B. findet sich eine Zusammenstellung dieser Verfahren in H. J. Laas et al., *J. Prakt. Chem.* **1994,** *336,* 185 ff.

Die überwiegende Mehrheit der vorstehend genannten Isocyanat-Modifizierungen wird durch den Einsatz verschiedener Katalysatoren wesentlich beschleunigt bzw. erst ermöglicht. Es hat sich dabei gezeigt, daß manche Katalysatoren gleichzeitig die Bildung mehrerer verschiedener Strukturtypen der vorstehend genannten Art katalysieren, was mitunter von Vorteil sein kann, wenn die Herstellung von Gemischen dieser unterschiedlich strukturierten Spezies angestrebt wird.

Allerdings ist häufig auch das Gegenteil der Fall, einerseits, da sich die gleichzeitige Anwesenheit von NCO-reaktiven Reaktionspartnern neben den Isocyanatgruppen des zu oligomerisierenden Isocyanates mitunter nachteilig auf die Oligomerisierung ersterer oder auf die Eigenschaften des erhaltenen Produktes auswirkt und andererseits, da die simultane Bildung anderer Strukturtypen als der der reinen NCO-Oligomerisate nicht immer glatt und vollständig unter den für die Bildung letzterer optimalen Bedingungen abläuft.

So ist die Anwesenheit von $CO_2$ beispielsweise bei der Tetraalkylammoniumhydroxidkatalysierten Trimerisierung von Hexamethylendiisocyanat (HDI) prohibitiv, um ein klares, qualitativ hochwertiges Produkt zu erhalten (DE-A 38 06 276).

Häufig werden Alkohole als Lösungsmittel für den Oligomerisierungskatalysator eingesetzt, die in der Regel, katalysiert durch letzteren, mit dem zu oligorimerisierenden Isocyanat über die Stufe des Urethans zum Allophanat reagieren. Mitunter wird auch bewußt mehr Alkohol zugemischt, sofern Produkte mit einem höheren Allophanatgehalt erwünscht sind. Diese Verbindungen, sofern sie auf der Basis monofunktioneller Alkohole hergestellt wurden, weisen eine niedrige Viskosität auf und sind, ähnlich den Uretdionen, als Reaktivverdünner zur Herstellung von Beschichtungsmitteln mit niedrigem VOC-Gehalt geeignet ('volatile organic compounds', d.h. die den Lack oder die Beschichtung bei der Aushärtung (gasförmig) verlassenden Anteile).

Allerdings verbleiben häufig bei der zur Trimerisierung simultanen Allophanatisierung der o.g. Alkohole, katalysiert durch typische Trimerisierungskatalysatoren wie Tetraalkylammoniumhydroxide, -carboxylate oder auch -carbonate, Rest-Urethane des entsprechenden Alkohols im Produkt und vermindern die NCO-Funktionalität und damit die Qualität der resultierenden Harze.

Hinzu kommt, daß die Vorteile von Uretdionen und Allophanaten bezüglich der Viskosität und des VOC-Gehaltes mit dem Makel einer niedrigen NCO-Funktionalität erkauft werden, was häufig zu Einbußen in der Qualität der Endprodukte, wie z.B. mangelhafte Lösungsmittelbeständigkeiten der aus ihnen gewonnenen Lackfilme fuhrt. Hierin liegt der besondere Vorteil der Trimeren begründet: auch das kleinste Oligomermolekül ist bereits ein Vernetzer (NCO-Funktionalität = 3).

Ein Beispiel für ein Verfahren, das ebenfalls mit dem Nachteil der Bildung von Gemischen aus erwünschtem und eher unerwünschtem Reaktionsprodukt behaftet ist, stellt die phosphinkatalysierte Oligomerisierung aliphatischer (Di)isocyanate dar. Abgesehen von der Luftempfindlichkeit der Alkylphosphine, ihrer unangenehmen physiologischen Eigenschaften und der Notwendigkeit, mit vergleichsweise hohen Konzentrationen bei der Oligomerisierung arbeiten zu müsssen, bilden sich in starker Abhängigkeit vom verwendeten Ausgangsisocyanat und den gewählten Reaktionsbedingungen in ihrer Zusammensetzung stark differierende Mischungen verschiedener Typen von Isocyanat-Oligomeren (Beispiel 1, vgl. auch H. J. Laas et al., *J. Prakt. Chem.* **1994**, *336,* 196 ff).

Niedrige Viskositäten des Produktes setzen Dimerisierung der Isocyanate voraus. Eine hohe NCO-Funktionalität dagegen wird durch Trimerisierung erreicht.

Es hat sich nun gezeigt, daß bei Phosphinkatalyse ein molares Verhältnis von Trimerisat, berechnet als $(NCO)_3$ Molmasse 126, zu Dimerisat, berechnet als $(NCO)_2$ Molmasse 84, von weniger als 5:1 nicht in Richtung höherer Trimerisatanteile verlagert werden kann, wenn man den Anfall von Uretoniminen

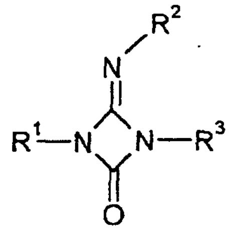

vermeiden will.

Die Uretonimine sind eine in der Chemie aliphatischer Polyisocyanate eher unerwünschte Verbindungsklasse, da sie bei noch niedrigerer Temperatur als die strukturell ähnlichen Uretdione in die Edukte dissoziieren, d.h. in (z.T. monomeres) Isocyanat und Carbodiimid 'zurückspalten'. Uretonimine können bei Zimmertemperatur im dynamischen Gleichgewicht mit Carbodiimiden vorliegen. Liefert ein monomeres (Di)isocyanat die zur Bildung einer Uretoniminstruktur aus einem beliebigen Carbodiimid nötige NCO-Gruppe, verursacht letzteres eine latente Restmonomerenproblematik, was aus arbeitshygienischen Gründen prohibitiv für einen breiten, gefahrlosen Einsatz entsprechender Produkte auf dem Polyurethan-Kunststoff- und Beschichtungsmittelsektor ist. Unter anderem aus diesem Grund wird in der (Patent)literatur auch immer wieder darauf hingewiesen, die phosphinkatalysierte Oligomerisierung monomerer Diisocyanate bei möglichst niedriger Temperatur durchzuführen (vgl. H. J. Laas et al., *J. Prakt. Chem*. **1994,** *336*, 196.).

Aus diesem Grund fehlt es nicht an Versuchen, zu qualitativ hochwertigen, rückspaltstabilen Produkten mit niedriger Viskosität zu gelangen, die trotzdem eine optimale, möglichst über 3 liegende, NCO-Funktionalität aufweisen.

Hierfür wird gegenwärtig mit sehr niedrigen Umsetzungsraten während der Trimerisierung gearbeitet, wobei als Hauptprodukt das aus drei Molekülen Diisocyanat aufgebaute Trimer erhalten wird. Dieses kann auch durch extraktive oder destillative Aufarbeitung von Oligomerenmischungen entsprechender Produkte, die mit einer höheren Umsetzungsrate aus monomerem Diisocyanat hergestellt wurden, abgetrennt werden. Weder das eine noch das andere Verfahren ist jedoch unter wirtschaftlichen Aspekten von Vorteil, da einerseits die niedrige Umsetzungsrate einen enormen Verlust in der Harzausbeute und einen großen Energieaufwand, hauptsächlich verursacht durch die im Anschluß an die Trimerisierung nötige Monomerabtrennung nach sich zieht und andererseits Extraktions- bzw. Destillationsverfahren, neben den Mehrkosten des Verfahrens, den Zwangsanfall höherviskoser Fraktionen verursachen.

Andererseits lassen sich niedrigviskose aliphatische Polyisocyanate mit optimaler Funktionalität auch durch alternative Aufbaureaktionen, z.B. durch Umsetzung silylierter Alkohole mit Isocyanatoalkansäurechloriden herstellen (Ch. Zwiener, L. Schmalstieg, M. Sonntag, K. Nachtkamp, und J. Pedain, *Farbe und Lack,* **1991**, 1052-1057 und darin zit. Literatur). Nachteilig hierbei ist, daß Isocyanatoalkansäurechloride technisch nicht zur Verfügung stehen und in ihrer Handhabung problematisch sein können. Das Verfahren ließe sich nur mit einem hohen technischen Aufwand realisieren, der durch die erwarteten Vorteile der Produkte, im wesentlichen die niedrige Viskosität der Polyisocyanate, nicht aufgewogen wird.

Aus den vorstehend genannten Ausführungen ist ersichtlich, daß die Entwicklung eines Verfahrens für die Isocyanatoligomerisierung, das die vorstehend zitierten Nachteile nicht in sich birgt, äußerst wünschenswert wäre.

Die prinzipielle Einsetzbarkeit von Hydrogen-Polyfluoriden zur NCO-Oligomerisierung wurde bereit in der nicht-vorveröffentlichten Patentanmeldung EP-A 0 778 299 erwähnt.

Der Erfindung lag die Aufgabe zugrunde, ein Verfahren zur Oligomerisierung von Di- bzw. Polyisocyanaten zu entwickeln, wobei möglicht niedrigviskose, rückspaltstabile Produkte erhalten werden sollen, die trotzdem eine hohe NCO-Funktionalität aufweisen.

Gegenstand der Erfindung ist ein Verfahren zur Herstellung von Polyisocyanaten, dadurch gekennzeichnet, daß Isocyanate der Formel (I)

$$(OCN\text{-}CH_2)X \qquad (I),$$

in welcher

X für gegebenenfalls verzweigte, gegebenenfalls cyclische, gegebenenfalls weitere Heteroatome sowie Heteroatomgruppierungen (N, O, S) sowie mindestens eine weitere NCO-Gruppe enthaltenden $C_3$-$C_{20}$-Substituenten steht,

durch Polymerisation in Gegenwart von Hydrogen-Polyfluoriden der Formel

$$M^{n+} n[F^{-} \cdot (HF)_m] \qquad (II),$$

in welcher
$m \geq 0{,}1$ ist und

M für ein n-fach-geladenes Kation bzw. eine summarisch n-fach-geladene Kationenmischung steht,

als Oligomerisierungskatalysator hergestellt werden.

Ein zur Durchführung des erfindungsgemäßen Verfahrens geeignetes Katalysatorsystem stellen Hydrogen (poly)fluoride der Formel (II) dar,
in welcher $m \geq 0{,}1$, bevorzugt $m \geq 0{,}5$, besonders bevorzugt $m \geq 1$, ist und

M für ein n-fach geladenes Kation und

n für die Zahl 1, 2, 3, 4, 5 oder 6 steht, bevorzugt 1 oder 2 bzw. eine summarisch n-fach-geladene Kationenmischung, bevorzugt vom Ammonium- bzw. Phosphoniumtyp, besonders bevorzugt vom Tetraalkylammoniumtyp, steht.

Es können als Katalysatoren zur Durchführung des erfindungsgemäßen Verfahrens Tetraorganyl-ammonium- bzw. -phosphoniumhydrogenpolyfluoride der Formel (III) eingesetzt werden:

$$R_4E^{+}[F^{-} \cdot (HF)_m] \qquad (III),$$

wobei

E für N oder P steht,

R für gleiche oder verschiedene, gegebenenfalls verzweigte, gegebenenfalls cyclische, gegebenenfalls substituierte (O, N, Halogen) aliphatische, araliphatische, sowie aromatische Reste mit jeweils 1 bis 25 C-Atomen steht und für m gilt:

m $\geq 0{,}1$, bevorzugt ist $m \geq 0{,}5$, besonders bevorzugt ist $m \geq 1$.

Bevorzugt werden als Katalysatoren zur Durchführung des erfindungsgemäßen Verfahrens Tetraalkylammoniumhydrogenpolyfluoride der Formel (III) eingesetzt, wobei E für N steht, R für gleiche oder verschiedene, gegebenenfalls verzweigte, gegebenenfalls substituierte (O, N, Halogen) (cyclo)aliphatische sowie araliphatische Reste mit jeweils 1 bis 20 C-Atomen steht und für m gilt:
$m \geq 0{,}1$, bevorzugt ist $m \geq 0{,}5$, besonders bevorzugt ist $m \geq 1$. Ein Beispiel für letztere sind Benzyltrimethylammoniumhydrogenpolyfluoride der Formel (IV):

$$C_6H_5CH_2(CH_3)_3N^{+}[F^{-} \cdot (HF)_m] \qquad (IV),$$

sowie Tetraalkylammoniumhydrogenpolyfluoride der Formel (V):

$$R_4N^{+}[F^{-} \cdot (HF)_m] \qquad (V),$$

wobei R für gleiche oder verschiedene, gegebenenfalls verzweigte, (cyclo)aliphatische Reste mit jeweils 1 bis 20 C-Atomen steht und
m wie folgt definiert ist: $m \geq 0{,}1$, bevorzugt ist $m \geq 0{,}5$, besonders bevorzugt ist $m \geq 1$.

Besonders bevorzugt werden als Katalysator Tetraalkyl-ammoniumhydrogenpolyfluoride der Formel (VI) eingesetzt:

$$R_3(R')N^+[F^- \cdot (HF)_m] \quad (VI),$$

wobei

R für gleiche oder verschiedene, gegebenenfalls verzweigte, aliphatische Reste mit jeweils 1 bis 15 C-Atomen steht, R' für, gegebenenfalls verzweigte, aliphatische Reste mit 1 bis 4 C-Atomen steht und

m für $m \geq 0{,}1$, bevorzugt $m \geq 0{,}5$, besonders bevorzugt ist $\geq 1$ steht.

Bedenkt man, daß in zahllosen Literaturbeispielen Säuren und Säurederivate zur sicheren 'Abstoppung' von Trimerisierungsreaktionen vorgeschlagen werden (H. J. Laas et al., *J. Prakt. Chem*. **1994,** *336*, 185 ff., dort weitere Zitate.), ist es außerordentlich überraschend, daß durch die Zugabe der Mineralsäure HF, beispielsweise zu quaternären Ammonium- oder Phosphoniumfluoriden, die katalytische Aktivität der Katalysatoren nicht zerstört wird!

Hydrogen(poly)fluoride sind teilweise kommerziell erhältlich oder lassen sich in einfacher Weise und in beliebiger Stöchiometrie durch Abmischen entsprechender Fluoride mit der gewünschten HF-Menge herstellen.

Es ist dabei belanglos, in welcher Form Fluorwasserstoff zugegeben wird. Dies kann in reiner Form, d.h. im flüssigen oder gasförmigen Aggregatzustand sein. Einfach handhabbar sind aber auch z.B. HF-Lösungen in protischen oder aprotischen organischen Lösungsmitteln. Kommerziell verfügbar und relativ handhabungssicher sind auch HF-Aminkomplexe, z.B. mit Aminen wie Triethylamin, Pyridin oder Melamin.

Im Gegensatz zum freien Fluorwasserstoff, der sich physiologisch unangenehm verhält, sind Hydrogenpolyfluoride unproblematisch. Auch ist durch die literaturbekannte Addition von HF an Isocyanate unter Bildung von Carbamoylfluoriden (vgl. G. D. Buckley, H. A. Piggott und A. J. E. Welch, *J. Chem. Soc.,* **1945**, 864-865) jeglichen eventuell vorhandenen HF-Resten in den erfindungsgemäßen Produkten das physiologische Gefährdungspotential genommen.

Der 'HF-Anteil' in den beschriebenen Katalysatorsystemen kann in weiten Grenzen variieren. D.h., es ist unwesentlich, ob es sich um definierte Monohydrogendifluoride (m = n = 1 in Formel I), Dihydrogentrifluoride (n = 1, m = 2 in Formel I) etc. handelt, die beispielsweise in Form ihrer Kaliumsalze mit der entsprechenden Stöchiometrie bekannt sind (Hollemann-Wiberg, Lehrbuch der Anorganischen Chemie, 91.-100. Aufl., W. de Gruyter Verlag, Berlin, New York, 1985, S. 408, Fußnote 50) oder um beliebige Mischungen letzterer mit überschüssigem Fluorid einerseits bzw. HF andererseits. Das optimale Design eines Polyfluorid-Katalysators kann sich je nach der gestellten Aufgabe und dem zu oligomerisierenden Isocyanat etwas unterscheiden, was in Vorversuchen leicht ermittelt werden kann.

Es ist zur Durchführung des erfindungsgemäßen Verfahrens unwesentlich, ob der Katalysator im zu oligomerisierenden Mono- bzw. Polyisocyanat löslich ist (Homogenkatalyse), oder nicht (Heterogenkatalyse). Auch können weitere Stoffe bzw. Stoffgemische bei der Katalyse zugesetzt werden, wie z.B. Amine, Alkohole, Phenole etc., Lösungsmittel für den Katalysator und/oder das Isocyanat, Antioxydanzien, sowie Matrizes zur adsorptiven oder kovalenten Bindung des Katalysators. Auch kann der zur Bildung der Hydrogen(poly)fluoride nötige Fluorwasserstoff separat, gegebenenfalls in gelöster Form, dem zu modifizierenden Isocyanat(gemisch) vor oder während der Trimerisierung zugefügt werden. Weiterhin können beliebige Substanzen, die Fluorwasserstoff unter den Katalysebedingungen liefern, zur Herstellung der erfindungsgemäßen Produkt(gemische) (mit)verwendet werden. So eignen sich beispielsweise beliebige Carbamoylfluoride als HF-Quelle, bevorzugt solche, die durch Addition von HF an das zu oligomerisierende (Di)isocyanat(gemisch) generiert werden können.

Nach dem erfindungsgemäßen Verfahren bei Einsatz von Polyfluoridkatalysatoren ist ganz allgemein eine breite Palette qualitativ hochwertiger und deshalb für den Polyurethansektor sehr wertvoller Polyisocyanate in einfacher Weise zugänglich geworden.

Auch stört die Gegenwart von Kohlendioxid, unabhängig davon, ob nur in Spuren oder in höherer Konzentration zugegen, die Durchführung des erfindungsgemäßen Verfahrens in keiner Weise (vgl. auch Beispiel 4).

Besonders überraschend ist dabei, daß die Polyfluorid-Katalyse insbesondere zur Herstellung Iminooxadiazindion-Gruppen ('asymmetrischer Trimerer') enthaltender Polyisocyanate der Formel (VII) geeignet ist

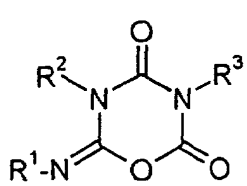

(VII),

in welcher

$R^1$, $R^2$, $R^3$ für gleiche oder verschiedene Reste, wie sie durch Entfernung einer Isocyanatgruppe aus monomeren bzw. oligomeren Isocyanaten der allgemeinen Formel $(OCN-CH_2)X$ entstehen, wobei X für einen, gegebenenfalls verzweigten, gegebenenfalls cyclischen, gegebenenfalls weitere Heteroatome sowie Heteroatomgruppierungen (NCO, O, Si, S) bzw. eine Verknüpfungsstelle zu einem NCO-Folgeprodukt enthaltenden $C_3$-$C_{20}$ Substituenten steht.

Diese Substanzklasse ist bisher wenig untersucht. Der erste Vertreter, das 3,5-Dimethyl-2-methylimino-4,6-diketo-1,3,5-oxadiazin ($R^1$= $R^2$= $R^3$ = Me in Abb. 2) wurde von Slotta und Tschesche durch Triethylphosphin-katalysierte Trimerisierung von Methylisocyanat neben dem isomeren Isocyanurat erhalten (*Chem. Ber*. **1927,** *60,* 295.). Es soll nach A. Etienne, G. Lonchambon, P. Girardeau und G. Durand, *C. R. Acad. Sci. Ser. C 277* **1973,** 795.) durch Trimerisierung mit demselben Katalysator in 1,2-Dichlorethan in besserer Ausbeute zugänglich sein. Weiterhin wurde das 5-Methyl-2-methylimino-3-phenyl-4,6-diketo-1,3,5-oxadiazin in 31%iger Ausbeute neben anderen Produkten bei der, technisch nicht relevanten, Reaktion von Methyliminokohlensäurediphenylester mit Tosylisocyanat als Nebenprodukt erhalten (E. Schaumann, J. Dietz, E. Kausch und G. C. Schmerse, *Chem. Ber*. **1987,** *120,* 339.). Strukturell mit den Iminooxadiazindionen verwandte Oxadiaziniumsalze werden, ebenfalls nur als Nebenprodukt, aus der Reaktion von Methyl- bzw. Isopropylisocyanat mit Antimonpentachlorid und Oxalylchlorid, Ethyloxalylchlorid bzw. Methylchloroformiat erhalten (A. Hamed, A. Ismail, M. G. Hitzler und J.C. Jochims, *J. prakt.Chem.* **1995**,*337,*385-390).

Wie überraschenderweise gefunden wurde und nicht aus Angaben der genannten Veröffentlichungen abgeleitet werden konnte, ist die auffälligste Besonderheit ihrer bei Zimmertemperatur flüssigen, NCO-polyfunktionellen Vertreter, wie sie beispielsweise aus HDI gemäß dem erfindungsgemäßen Verfahrens zugänglich sind, die signifikant niedrigere Viskosität im Vergleich zu Verbindungen vom isomeren Isocyanurat-Strukturtyp. Analoges gilt für die Schmelz- bzw. Lösungsviskosität der bei Zimmertemparatur festen Polyisocyanate beispielsweise auf Basis cycloaliphatischer Diisocyanate (vgl. auch Beispiel 8).

Es finden sich sehr vereinzelt Hinweise auf die Bildung von Iminooxadiazindionen als untergeordnete Nebenreaktion bei der katalysierten Oligomerisierung aliphatischer Isocyanate. Wie bereits weiter oben vermerkt, waren die physikalischen und chemischen Eigenschaften von Polyisocyanaten mit Iminooxadiazindionstruktur bisher völlig unbekannt.

So sollen sich bei der Phosphin-katalysierten Uretdionbildung ('Dimerisierung') aliphatischer Diisocyanate "bei höheren Temperaturen und vor allem bei längerer Temperaturbelastung und geringer Katalysatorkonzentration ... neben Isocyanuraten in wachsender Menge auch andere Nebenprodukte, wie Alkylimino-dialkyloxadiazindione, Carbodiimide und Uretonimine bilden" (DE-A 16 70 720, S. 5, Zeilen 1-5). Genauere Angaben über den Anteil dieser Nebenprodukte finden sich in der genannten Veröffentlichung allerdings nicht.

Wie bereits eingangs erwähnt, haben unsere Untersuchungen hierzu gezeigt, daß weder eine gezielte Herstellung von Dimeren (Uretdionen) noch von Trimeren (Isocyanuraten, Iminooxadiazindionen) durch Phosphinkatalyse gelingt und der Anfall von Carbodiimiden/Uretoniminen bei höherer Temperatur unvermeidbar ist. Der molare Anteil an Iminooxadiazindion im Produkt hingegen, insbesondere aber das Verhältnis von Isocyanurat zu Iminooxadiazindion, bleibt unabhängig von den Reaktionsbedingungen nahezu konstant (vgl. Beispiel 1). Die in der DE-A 16 70 720 getroffene Aussage ist also nur teilweise korrekt.

Daß Carbodiimide bzw. Uretonimine eine in der Chemie aliphatischer Polyisocyanate eher unerwünschte Verbindungsklasse sind, wurde bereits weiter oben ausgeführt.

In der DE-A 39 02,078 wird ein Verfahren beschrieben, nach dem durch Trimerisierung (cyclo)aliphatischer Diisocyanate mit Hilfe von quaternären Ammonium- bzw. Phosphoniumfluoriden in Gegenwart von Kohlendioxid neben Oxadiazintrionen und Isocyanuraten auch in untergeordnetem Maße, (S. 4, Zeilen 51-52), Iminooxadiazindione mitentstehen. Deren Anteil liegt bezogen auf den Trimerisatanteil nicht über 25 mol-%. Dasselbe Katalysatorsystem sowie dessen Verwendung zur Herstellung von Isocyanuratgruppen aufweisenden Polyisocyanaten wird in der EP-A 03 55 479 beschrieben. Es findet sich allerdings weder ein Verweis auf die anteilige Bildung von Iminooxadiazindionen, noch auf eine niedrigere Viskosität der Verfahrensprodukte in dieser Patentschrift.

Es war mithin unbekannt, daß die Verwendung von Fluoriden ganz allgemein, d.h. auch in Abwesenheit von $CO_2$, die Bildung von Iminooxadiazindionen, verglichen mit Verfahren des Standes der Technik, etwas stärker begünstigt. Die Ausführungen der DE-A 39 02 078 legen eher den Schluß nahe, daß die Iminooxadiazindionbildung an die Präsenz von $CO_2$ während der Reaktion und mithin an die simultane Bildung von Oxadiazintrionen (aus 2 mol NCO und einem mol $CO_2$) geknüpft ist.

Wie die Ausführungsbeispiele der EP-A 0 355 479, die sich auf HDI-Trimerisierungen beziehen, zeigen, lassen sich durch die Verwendung der beschriebenen Fluorid-Katalysatorsysteme zur HDI-Trimerisierung bei einer Harzausbeute von 22, 60 bzw. 25 % dynamische Viskositäten, gemessen bei 23°C, von 2000, 35 000 bzw. 2500 mPa·s erzielen. Die nach der Lehre der DE-A 3806276 durch Katalyse mit quaternären Ammoniumhydroxiden erhältlichen Isocyanurat-Polyisocyanate weisen bei HDI-Trimerisatausbeuten von 32 bzw. 52 % Viskositätswerte von ca. 1500 bzw. 9 800 mPa·s bei 23°C auf (vgl. DE-A 3806276, Beispiele 11 bzw. 9). Demzufolge stellen die nach der Lehre der EP-A 0 355 479 durch Fluoridkatalyse erhaltenen HDI-Trimerisate bezüglich der Viskosität keine Verbesserung dar. Ein Zusammenhang zwischen Iminooxadiazindionanteil im Trimerengemisch, Fluoridkatalyse und Viskosität der Harze war mithin für den Fachmann nicht ersichtlich.

Es ist deshalb überraschend, daß erfindungsgemäß eine signifikante Erhöhung des Iminooxadiazindionanteils in einer Trimerisatmischung einerseits möglich ist und andererseits zu einer drastischen Absenkung der Viskosität dieser Produkte führt.

Wie eigene Untersuchungen zur HDI-Trimerisierung mit vorbeschriebenen Fluorid-Katalysatorsystemen ergaben, ist auch bei Variation der Herstellungsbedingungen des Polyisocyanates (Cokatalysator, Temperatur, Kation etc.) der Iminooxadiazindionanteil im Trimerengemisch nie größer als 25 %, in der Regel liegt er unter 20 % (Beispiel 2). Ganz allgemein ist es nahezu unmöglich, Aussagen bezüglich der Viskosität einer bestimmten Verbindung oder Verbindungsklasse aus Analogieschlüssen aus der Struktur oder auch der NCO-Funktionalität von anderen Verbindungen bzw. Verbindungstypen herleiten zu wollen.

Zwar weisen Polyisocyanate mit niedrigerer NCO-Funktionalität wie z.B. Uretdione oder Allophanate (sofern sie auf der Basis monofunktioneller Alkohole hergestellt wurden) in der Regel eine niedrigere Viskosität als höherfunktionelle Vertreter, wie z.B. Isocyanurate auf, dieser Zusammenhang kann sich aber auch umkehren. So weist z.B. das 1,3,5-Tris(6-isocyanatohexyl)isocyanurat mit ca. 700 mPa·s bei 23°C eine erheblich geringere dynamische Viskosität als das strukturell verwandte, aber nur NCO-difunktionelle 3,5-Bis(6-isocyanatohexyl)-1-oxadiazintrion auf, dessen Viskosität bei 23°C ca. 1200 mPa·s beträgt (Beispiel 3).

Ein weiterer Vorteil der erfindungsgemäß hergestellben Polyisocyanate mit Iminooxadiazindionstruktur ist darin zu sehen, daß sie selbst bei längerer thermischer Belastung keine Rückspalttendenz in die zugrundeliegenden monomeren Aufbaukomponenten, in der Regel Diisocyanate, aufweisen. So lassen sich selbst so hochsiedende Verbindungen wie das N,N',N''-Tris(6-isocyanatohexyl)iminooxadiazindion ohne Zersetzung oder Umlagerung zum isomeren Isocyanurat zu erleiden sowohl destillativ als auch extraktiv von höhermolekularen Produkten aus den erfindungsgemäßen HDI-Trimerenmischungen abtrennen. Hierbei resultieren Produkte, die die literaturbekannte Viskosität des 1,3,5-Tris-(6-isocyanatohexyl)isocyanurates von 700 mPa·s bei 23°C wesentlich unterschreiten (Beispiel 6). Das N, N', N''-Tris-(6-isocyanatohexyl)iminooxadiazindion ist mithin das am niedrigsten viskose, NCO-trifunktionelle Oligomer des Hexamethylendiisocyanates. Entsprechendes gilt für asymmetrische Trimere (Iminooxadiazindione) anderer (Di)isocyanate.

Zur Durchführung des erfindungsgemäßen Verfahrens werden Katalysatorkonzentrationen, bezogen auf eingesetztes (Poly)isocyanat bzw. auf eingesetzte (Poly)isocyanatmischung und die Masse des Fluoridions, von weniger als 0,1 %, bevorzugt weniger als 0,05 % eingesetzt. Sofern primäre NCO-Gruppen enthaltende, linearaliphatische Diisocyanate wie z.B. HDI oligomerisiert werden sollen, können bezogen auf die Masse des Fluoridanions und das eingesetzte Diisocyanat auch weniger als 50 ppm genügen.

Das erfindungsgemäße Verfahren kann in einem Temperaturbereich von 0°C bis +250°C, bevorzugt 20 bis 180°C, besonders bevorzugt 40 bis 120°C, in kondensierter Phase oder in der Gasphase, wahlweise unter quantitativer Umsetzung der beteiligten Isocyanatgruppen des Ausgangsisocyanates bzw. der Ausgangsisocyanatmischung erfolgen oder bei beliebigen Umsetzungsgraden, bevorzugt nachdem 10 bis 90 %, besonders bevorzugt 20 bis 60 %, des eingesetzten monomeren Diisocyanates umgesetzt wurden, unterbrochen werden.

Zur Abstoppung der Reaktion, d.h. zur Inaktivierung des Katalysatorsystems, bieten sich prinzipiell alle vorbeschriebenen Methoden des Standes der Technik an, wie z.B. die Zugabe (unter)stöchiometrischer Mengen an Säuren oder Säurederivaten (z.B. Benzoylchlorid, saure Ester Phosphor- oder Schwefel enthaltender Säuren, diese Säuren selbst etc., nicht jedoch HF), adsorptive Bindung des Katalysators und anschließende Abtrennung durch Filtration, thermische Desaktivierung usw.

Gegenüber der Phosphinkatalyse besteht bei der erfindungsgemäßen Polyfluoridkatalyse der entscheidende Vorteil daß, abgesehen von der erheblich geringeren, zur Oligomerisierung erforderlichen Katalysatorkonzentration auch bei hoher Temperatur keine Tendenz zur Carbodiimidisierung und/oder Uretoniminbildung zu beobachten ist.

Verglichen mit der Katalyse durch quaternäre Ammoniumhydroxide, -carboxylate, -carbonate oder auch -fluoride erweist sich das erfindungsgemäße Verfahren unter mehreren Aspekten als vorteilhaft. Es kann in der Homogenkatalyse mit erheblich konzentrierteren Katalysatorlösungen bis hin zu reinen Polyfluoriden, so diese flüssig oder im zu oligomerisierenden Isocyanat löslich sind, gearbeitet werden. Nebenreaktionen mit dem Katalysatorlösungsmittel spielen mithin kaum eine Rolle. Die bei Verwendung der obengenannten Katalysätortypen häufig zu beobachtenden, schwer beeinflußbaren Exothermieerscheinungen und Trübungen infolge spontaner Übervernetzungen treten kaum auf Die Lagerstabilität der Polyfluorid-Katalysatoren bzw. -lösungen an sich, auch bei erhöhter Temperatur, ist ebenfalls verbessert.

Nach einer besonderen, kontinuierlich arbeitenden Ausführungsform des erfindungsgemäßen Verfahrens kann die Oligomerisierung in einem Rohrreaktor vorgenommen werden. Hierbei profitiert man ebenfalls von der geringeren Neigung der Polyfluoridkatalysatoren, trotz Applikation in hochkonzentrierter Lösung bzw. als reiner Wirkstoff, spontan Gelteilchen im Produkt zu bilden.

Zur Durchführung des erfindungsgemäßen Verfahrens können alle (Di)isocyanate der allgemeinen Formel $(OCN-CH_2)X$ (I) in reiner Form oder als beliebige Mischungen untereinander verwendet werden.

In Formel (I) steht X für einen gegebenenfalls verzweigten, gegebenenfalls cyclischen, gegebenenfalls weitere Heteroatome sowie Heteroatomgruppierungen (N, O, S) sowie mindestens eine weitere NCO-Gruppe enthaltenden $C_3$-$C_{20}$ Substituenten.

Einige der in Frage kommenden Substituenten X sind beispielhaft genannt (jeweils alle Regio- und Stereoisomeren): Mono- (Di, **T**ri, usw.)-Isocyanato-propyl, -butyl, -pentyl (d.h. $(OCN-CH_2)X$ = z.B.: Hexamethylendiisocyanat (HDI), 2-Methylpentan-1,5-diisocyanat, etc.), -hexyl, -octyl (d.h. $(OCN-CH_2)X$ = z.B. 4-Isocyanatomethyl-1,8-octandiisocyanat), -nonyl, -decyl, -alkoxyalkyl, -cyclohexyl, -(methyl)cyclohexyl (d.h. $(OCN-CH_2)X$ = z.B.: 3(4)-Isocyanatomethyl-1-methylcyclohexylisocyanat (IMCI)), -(dimethyl)cyclohexyl, (trimethyl)cyclohexyl (d.h. $(OCN-CH_2)X$ = z.B. Isophorondiisocyanat (IPDI)), -ethylcyclohexyl, -propylcyclohexyl etc., (-methyl)phenyl, (-methyl)cyclohexyl sowie -methyl-isocyanatocyclohexyl. Hierbei ist es belanglos, nach welchen Verfahren die vorstehend genannten (Poly)isocyanate generiert werden, d.h. mit oder ohne Verwendung von Phosgen.

Es kann weiterhin von Vorteil sein, Gemische bestimmter (Poly)isocyanate im erfindungsgemäßen Verfahren einzusetzen, beispielsweise um dem Anforderungsprofil des jeweiligen Produktes bzw. Produktgemisches optimal zu entsprechen. So werden in vielen Anwendungen, beispielsweise bei der Automobil(erst)lackierung, Gemische von Isocyanurat-Polyisocyanaten auf Basis, gegebenenfalls verzweigter, linearaliphatischer, z.B. HDI, einerseits und cycloaliphatischer Diisocyanate, z.B. IPDI oder $H_{12}$MDI (Desmodur® W-Handelsprodukt der Bayer AG), andererseits eingesetzt. Diese Gemische werden in der Regel durch nachträgliches Abmischen reiner Isocyanurat-Polyisocyanate auf Basis von Diisocyanaten des einen mit denen des anderen Typs hergestellt. Es kann aber auch von Vorteil sein, sie durch simultane Mischtrimerisation aus dem entsprechenden Gemisch der monomeren Komponenten herzustellen (EP-A 0 047 452). Da aber insbesondere Isocyanurat-Polyisocyanate des Standes der Technik auf Basis cycloaliphatischer Diisocyanate fest sind und zuweilen eine derart hohe Schmelzviskosität aufweisen, daß eine Monomerenabtrennung durch (Dünnschicht)destillation erhebliche Schwierigkeiten bereitet, bedarf es zu ihrer Verarbeitung der Verwendung von Lösungsmitteln und mitunter auch von Fließhilfsmitteln bei der Dünnschichtdestillation. Will man keine allzu großen Einbußen in Umsetzungsgrad (Harzausbeute) und NCO-Funktionalität bei der Herstellung von Isocyanurat-Polyisocyanaten hinnehmen, sind Lösungskonzentrationen um 70 % an Isocyanurat-Polyisocyanat auf Basis cycloaliphatischer Diisocyanate bei dynamischen Viskositäten, gemessen bei 23°C, zwischen 1000-10 000 000 mPa·s marktüblicher Stand der Technik.

Trimerisiert man hingegen Gemische linearaliphatischer, z.B. HDI, und cycloaliphatischer Diisocyanate, z.B. IPDI, gemäß dem erfindungsgemäßen Verfahren unter (teilweiser) Iminooxadiazindionbildung, erhält man auch bei Zimmertemperatur fließfähige Produkte (Viskosität bei 23°C unter 100 000 mPa·s) die zudem in Lösung eine drastisch schnellere Viskositätsabnahme bei steigendem Lösungsmittelanteil aufweisen, als Produkte entsprechender Zusammensetzung (NCO-Funktionalität, Diisocyanatbasis, mittleres Molekulargewicht) des Standes der Technik (Beispiel 7).

Die nach dem erfindungsgemäßen Verfahren erhältlichen Produkte bzw. Produktgemische stellen mithin vielseitig verwendbare Ausgangsmaterialien zur Herstellung von, gegebenenfalls geschäumte(n), Kunststoffe(n) sowie Lacken, Beschichtungsmitteln, Klebstoffen und Zuschlagstoffen dar. Insbesondere zur Herstellung von, gegebenenfalls wasserdipergierbaren Ein- und Zweikomponenten-Polyurethanlacken eignen sie sich, gegebenenfalls in NCO-blockierter Form, aufgrund ihrer im Vergleich zu (überwiegend) Isocyanurat-Polyisocyanat basierenden Produkten verringerten Lösungs- sowie Schmelzviskosität bei ansonsten gleich hohem bzw. verbessertem Eigenschaftsprofil. So sind die erfindungsgemäßen Verfahrensprodukte auf HDI-Basis auch in hoher Verdünnung in Lacklösungsmitteln stabiler gegen das Auftreten von Ausflockungen bzw. Trübungen, als entsprechende, im wesentlichen Isocyanuratgruppen enthaltende Produkte des Standes der Technik. Ihre Resistenz gegenüber der Einwirkung von Luftfeuchtigkeit (Hautbildung im offenen Gebinde) ist ebenfalls gegenüber den Isocyanurat-basierenden Produkten verbessert.

Sie können rein oder in Verbindung mit anderen Isocyanatderivaten des Standes der Technik, wie z.B. Uretdion-, Biuret-, Allophanat-, Isocyanurat- und/oder UrethanGruppen enthaltenden Polyisocyanaten, deren freie NCO-Gruppen gegebenenfalls mit Blockierungsmitteln desaktiviert wurden, eingesetzt werden.

## Beispiele

Alle Prozentangaben sind, soweit nicht anders vermerkt, Gewichtsprozente.

Mol-% Angaben wurden NMR-spektroskopisch ermittelt und beziehen sich immer, so nicht anders ausgewiesen, auf die Summe der NCO-Folgeprodukte. Die Messungen erfolgten auf dem Gerät DPX 400 der Fa. Brucker an ca. 5 %igen ($^1$H-NMR) bzw. ca. 50 %igen ($^{13}$C-NMR) Proben in trockenem $CDCl_3$ bei einer Frequenz von 400 MHz ($^1$H-NMR) bzw. 100 MHz ($^{13}$C-NMR). Als Referenz für die ppm-Skale wurden geringe Mengen von Tetramethylsilan im Lösungsmittel mit einer $^1$H-chem. Verschiebung von 0 ppm ($^1$H-NMR) bzw. das Lösungsmittel selbst ($CDCl_3$) mit

einer Verschiebung von 77,0 ppm ($^{13}C$-NMR) gewählt. Daten für die chemische Verschiebung der in Frage kommenden Verbindungen wurden der Literatur entnommen (vgl. D. Wendisch, H. Reiff und D. Dieterich, Die Angewandte Makromolekulare Chemie 141, 1986, 173-183 und darin zit. Lit) bzw. durch Vermessung von Modellsubstanzen gewonnen. Das in Anlehnung an das von Slotta und Tschesche in *Chem. Ber.* **1927,** *60*, 295 beschriebene Verfahren aus Methylisocyanat unter Katalyse mit ca. 3 % Tri-n-butylphosphin in ca. 70 %iger Ausbeute zugängliche 3,5-Dimethyl-2-methylimino-4,6-diketo-1,3,5-oxadiazin weist folgende NMR-chem. Verschiebungen auf (in ppm): 3,09; 3,08 und 2,84 ($^{1}H$-NMR, $C\underline{H}_3$) bzw. 148,3; 144,6 und 137,3 ($^{13}C$-NMR, $\underline{C}$=O/$\underline{C}$=N). Die erfindungsgemäßen Produkte mit Iminooxadiazindionstruktur haben sehr ähnliche $^{13}C$-NMR chem. Verschiebungen der $\underline{C}$=O/$\underline{C}$=N Atome und sind zweifelsfrei als solche von anderen Isocyanat-Folgeprodukten zu unterscheiden. HDIbasierende Oxadiazintrione geben sich im $^{13}C$-NMR Spektrum u.a. durch zwei Signale bei 147,8 und 143,9 ppm für die Ring-$\underline{C}$=O-Atome zu erkennen (vermessen wurde das Handelsprodukt Baymicron® Oxa WM 06 der Bayer AG).

Die dynamischen Viskositäten wurden bei 23°C mit dem Viskosimeter VT 550 der Fa. Haake bestimmt. Durch Messungen bei unterschiedlichen Schergeschwindigkeiten wurde sichergestellt, daß das Fließverhalten der beschriebenen erfindungsgemäßen Polyisocyanatmischungen wie auch das der Vergleichsprodukte dem idealer Newtonscher Flüssigkeiten entspricht. Die Angabe der Schergeschwindigkeit kann deshalb entfallen.

Die Bestimmung der Restmonomergehalte erfolgte gaschromatographisch.

Alle Reaktionen wurden unter einer Stickstoffatmosphäre durchgeführt.

**Beispiel 1:** Vergleichsbeispiel zur Phosphinkatalyse (nicht erfindungsgemäß)

Jeweils 200 g (1,19 mol) frisch destilliertes HDI werden bei 60°C zunächst im Vakuum (0,1 mbar) zur Entfernung gelöster Gase 1h gerührt, anschließend mit trockenem Stickstoff belüftet und bei:

a) 60°C,
b) 120°C bzw.
c) 180°C

mit jeweils 3 g (14,8 mmol) Tri-n-butylphosphin (Fa. Acros) versetzt und bis zum Erreichen des in Tab. 1 angegebenen Brechungsindex' der Rohlösung unter einer Stickstoffatmosphäre umgesetzt. Anschließend wird durch Zugabe von jeweils 4 g (26 mmol) p-Toluolsulfonsäuremethylester (Fa. Merck) und einstündiges Nachrühren bei 80°C die Weiterreaktion sicher unterbunden. Die Rohprodukte werden anschließend durch Dünnschichtdestillation bei 120°C/0,1 mbar in einem Kurzwegverdampfer vom nicht umgesetzten Monomer befreit. Anschließend wird die Produktzusammensetzung NMR-spektroskopisch und der Restmonomergehalt gaschromatographisch bestimmt. Letzterer wird nach 3-wöchiger Lagerung bei 20-25°C (Zimmertemperatur) und anschließender 2-wöchiger Lagerung bei 50°C im Trockenschrank nochmals bestimmt. Sämtliche Analysenergebnissse sind in Tabelle 1 zusammengestellt,

Tab. 1

| Resultate der Tributylphosphin-katalysierten HDI-Oligomerisierung bei verschiedenen Temperaturen | | | | | | | | | | |
|---|---|---|---|---|---|---|---|---|---|---|
| Versuch | $n_D^{20}$ (Stop) | Harzausbeute [%] | Viskosität [mPa·s] | Restmonomergehalt [%] | Carbodiimide/ Uretonimine [mo(-%] | Uretdione [mol-%] | Isocyanurate [mol-%] | Imino-oxadiazin- dione [mol-%] | molares Verhältnis von Trimerisaten[3] zu Dimerisaten (Uretdionen) | molares Verhältnis von Isocyanuraten zu Iminooxadiazindionen |
| 1a | 1,4732 | 46,3 | 240 | 0,1/0,1/0,2[1] | n.n.[2] | 69 | 22 | 9 | 0,45 | 2,4 |
| | | | | | | | | | | |
| 1b | 1,4731 | 40,0 | 1250 | 0,3/0,3/0,4[1] | 3 | 28 | 47 | 22 | 2,5 | 2,1 |
| | | | | | | | | | | |
| 1c | 1,4768 | 39,4 | 5200 | 2,7/3,8/5,4[1] | 54 | 4 | 30 | 12 | 10,5 | 2,5 |

1) Restmonomergehalt nach -Aufarbeitung/-3-wöchiger Lagerung bei 20-25°C Zimmertemperatur)/-weiterer 2-wöchiger Lagerung bei 50°C,

2) n.n. = nicht nachweisbar,

3) Trimerisate = Summe von Isocyanuraten und Iminooxadiazindionen

**Beispiel 2:** Vergleichsbeispiel zur Fluoridkatalyse (nicht erfindungsgemäß)

Jeweils 200 g (1,19 mol) frisch destilliertes HDI werden bei 60°C zunächst im Vakuum (0,1 mbar) zur Entfernung gelöster Gase 1h gerührt, anschließend mit trockenem Stickstoff belüftet und wie folgt weiter behandelt:

a) es werden bei 80°C ca. 900 ppm, bezogen auf Katalysator der $C_8$-Stöchiometrie und eingesetztes HDI, entsprechend ca. 44 ppm Fluorid ($F^-$), einer ca. 8%igen Katalysatorlösung von Methyl(trialkyl)ammoniumfluorid, Alkyl steht für $C_8$-$C_{10}$, in 2-Ethyl-1,3-hexandiol (hergestellt wie in DE-A 39 02 078, Beispiel 1 beschrieben) zugegeben, wobei die Temperatur bis auf maximal 105°C ansteigt und bis zum Erreichen eines NCO-Gehaltes von 41,2 % gerührt. Anschließend wird durch Zugabe von 0,9 g Phosphorsäuredi-n-butylester abgestoppt, eine weitere Stunde bei 60°C gerührt und anschließend durch Dünnschichtdestillation in einem Kurzwegverdampfer (120°C/0,1) mbar vom nicht umgesetzten Monomer befreit. Anschließend wird wie in Beispiel 1 beschrieben analysiert (vgl. Tabelle 2);

b) es wird verfahren wie bei a) angegeben, mit dem Unterschied, daß als Katalysator ca. 110 ppm, bezogen auf Katalysator der Stöchiometrie ($Me_4N^+F^-$ x $H_2O$] und eingesetztes HDI, entsprechend ca. 18 ppm Fluorid ($F^-$), einer ca. 5 %igen Lösung von Tetramethylammoniumfluorid-Tetrahydrat (Fa. Aldrich) in n-Butanol verwendet werden, die Reaktion im Temperaturbereich von 60 - 70°C durchgeführt wird, bis zu einem NCO-Gehalt von 39,1 % trimerisiert und durch Zugabe von 0,132 g Phosphorsäuredi-n-butylester abgestoppt wird;

c) es wird verfahren wie bei a) angegeben, mit dem Unterschied, daß als Katalysator ca. 190 ppm, bezogen auf Katalysator der Stöchiometrie [$Et_4N^+$ F- x $H_2O$] und eingesetztes HDI, entsprechend ca. 22 ppm Fluorid ($F^-$), einer ca. 8 %igen Lösung von Tetraethylammoniumfluorid-Hydrat (Fa. Aldrich) in n-Butanol verwendet werden, im Temperaturbereich von 70-150°C trimerisiert wird bis der NCO-Gehalt 39,9 % beträgt und durch Zugabe von 0,312 g Phosphorsäuredi-n-butylester abgestoppt wird;

d) es wird verfahren wie bei a) angegeben, mit dem Unterschied, daß als Katalysator ca. 160 ppm, bezogen auf Katalysator der Stöchiometrie [$Bz(Me)_3N^+$ $F^{-x}$ $H_2O$] und eingesetztes HDI, entsprechend ca. 16 ppm Fluorid ($F^-$), einer ca. 5 %igen Lösung von Benzyltrimethylammoniumfluorid-Hydrat (Fa. Aldrich) in 2-Ethyl-1,3-hexandiol (Fa. Janssen) verwendet werden, bis zu einem NCO-Gehalt von 35,1 % trimerisiert und durch Zugabe von 0,03 g Phosphorsäuredi-n-butylester abgestoppt wird.

Wie Tabelle 2 zu entnehmen ist, liegt der molare Iminooxadiazindionanteil im Trimerisatgemisch (Summe aus Isocyanurat und Iminooxadiazindion) immer weit unter 30 %.

Tab. 2

| Die Resultate der Fluorid-katalysierten Trimerisierung von HDI | | | | | | | |
|---|---|---|---|---|---|---|---|
| Versuch | Iminooxadiazindione **A** [mol-%] | Isocyanurate **B** [mol-%] | Urethane [mol-%] | Allophanate [mol-%] | Uretdione [mol-%] | Carbodiimide/ Uretonimine [mol-%] | Verhältnis **A:B** |
| 3a | 14 | 68 | n.n.[1)] | 13 | 5 | n.n.[1)] | 17:83 |
| | | | | | | | |
| 3b | 18 | 74 | n.n.[1)] | 5 | 3 | n.n.[1)] | 20:80 |
| | | | | | | | |
| 3c | 21 | 69 | 2 | 5 | 3 | n.n.[1)] | 23:77 |
| | | | | | | | |
| 3d | 15 | 69 | 2 | 10 | 4 | n.n.[1)] | 18:82 |

[1)] n.n. = nicht nachweisbar

**Beispiel 3** (Vergleichsbeispiel)

Jeweils 1500 g eines

a) HDI-Isocyanurat-Polyisocyanates mit einem NCO-Gehalt von 23,5 % und einer Viskosität von 1380 mPa·s, hergestellt nach der Lehre der DE-A 38 06 276, bzw.

b) eines HDI-Oxadiazintrion-Polyisocyanates mit einem NCO-Gehalt von 22,5 % und einer Viskosität von 2560 mPa·s, hergestellt nach der Lehre der DE-A 16 70 666 (Handelsprodukt Baymicron® Oxa WM 06 der Bayer AG), werden bei einem Druck von 0,05 mbar und einer Temperatur des Heizmediums von 220°C der Dünnschichtdestillation in einem Kurzwegverdampfer unterworfen.

Dabei gehen

a) 364 g bzw.

b) 1092 g Destillat über, die anschließend jeweils bei 120°C/0,05 mbar von monomerem HDI durch erneute Dünnschichtdestillation befreit werden. Die so gereinigten, farblosen Flüssigkeiten weisen eine Viskosität von:

a) 700 ± 10 mPa·s bei 23°C bzw.

b) 1200±20 mPa·s bei 23°C auf und bestehen nach Ausweis kombinierter analytischer Methoden (IR, NMR, GPC) zu mindestens 98 % aus

a) dem idealen, d.h. aus drei Molekülen HDI aufgebauten, Isocyanurat-Trimeren des Hexamethylendiisocyanates (1,3,5-Tris(6-isocyanatohexyl)isocyanurat) bzw.

b) dem aus zwei Molekülen HDI und einem Molekül $CO_2$ aufgebauten 3,5-Bis(6-isocyanatohexyl)-1-oxadiazintrion.

Die unter a) aufgeführten Befunde stehen mithin völlig in Übereinklang mit literaturbekannten Daten (vgl. WO-A 93/07 183, die in den dort angegebenen Beispielen zitierten Viskositäten wurden bei 25°C an weniger reinen "Ideal-Isocyanurat"-Fraktionen gemessen). Für das Oxadiazintrion stehen keine Vergleichsangaben aus der Literatur zur Verfügung.

**Beispiel 4** (erfindungsgemäß)

a) Herstellung des Katalysators

Zu 2,3 g (49 mmol) Kaliumfluorid, gelöst in 50 g Methanol, welches 1 % Wasser enthält, werden 16 g (47,6 mmol) Aliquat® 336 (Fa. Fluka), gelöst in 50 g Methanol der selben Qualität wie oben erwähnt, gegeben, bei Zimmertemperatur 24 h gerührt und anschließend filtriert. Anschließend werden erneut 2,3 g Kaliumfluorid zum Filtrat gegeben und weitere 24 h bei Zimmertemperatur gerührt. Die anorganischen Salze werden anschließend abfiltriert, und das Filtrat wird bei Zimmertemperatur im Vakuum (0,1 mbar) mit einem Rotationsverdampfer aufkonzentriert, bis kein Lösungsmittel (Methanol) mehr übergeht.

Der Fluoridgehalt der verbleibenden, klaren, leicht gelben Flüssigkeit beläuft sich auf 2,7 % (mittels Fluoridionensensitiver Elektrode bestimmt). Der Restchlorgehalt wird elementaranalytisch zu 0,2 % bestimmt.

0,95 g (14,2 mmol) einer 30 %igen HF-Lösung in iso-Propanol werden tropfenweise unter Rühren und Kühlen (0°C) zu 10 g (14,2 mmol Fluorid) der obengenannten quaternären Ammoniumfluoridlösung gegeben. Der Fluoridgehalt (formales $F^-$, nicht Gesamt-Fluor(!)) der fertigen Katalysatorlösung beläuft sich mithin auf 2,5 %, entsprechend ca. 50 bis 60 % Katalysator der Formel $R_3(Me)N^+ [HF_2]^-$, R = lt. Angabe des Fluka-Katalogs, $C_8$-$C_{10}$, wobei $C_8$ überwiegt. Diese Lösung wird als Katalysator für die weiter unten beschriebenen Trimerisierungsreaktionen verwendet.

b) Trimerisierung

200 g (1,19 mol) frisch destilliertes HDI werden bei Zimmertemperatur (20 bis 25°C) durch einstündiges Durchleiten eines lebhaften $CO_2$-Stromes mit Kohlendioxid gesättigt, auf 60°C erwärmt und anschließend mit ca. 400 ppm, bezogen auf Katalysator der $C_8$-Stöchiometrie und eingesetztes HDI, entsprechend ca. 19 ppm Fluorid ($F^-$) der obengenannten Katalysatorlösung versetzt, wobei die Temperatur bis auf maximal 80°C ansteigt und bis zum Erreichen

eines NCO-Gehaltes von 41,2 % gerührt. Anschließend wird durch Zugabe von 0,9 g Phosphorsäuredi-n-butylester abgestoppt, eine weitere Stunde bei 60°C gerührt und schließlich durch Dünnschichtdestillation in einem Kurzwegverdampfer (120°C/0,1) mbar vom nicht umgesetzten Monomer befreit. Man erhält ein farbloses, klares Harz mit einem NCO-Gehalt von 22,8 % das lt. NMR-spektroskopischer Analyse folgende Zusammensetzung aufweist: ca. 4 mol-% Uretdione, ca. 6 mol-% Oxadiazintrione, ca. 54 mol-% Isocyanurate sowie ca. 36 mol-% Iminooxadiazindione. Carbodiimide bzw. Uretonimine waren nicht nachweisbar.

Dieses Beispiel zeigt, daß nach dem erfindungsgemäßen Verfahren, im Gegensatz zur HDI-Trimerisierung mit quaternären Ammoniumhydroxiden (vgl. DE-A 38 06 276) problemlos auch in Gegenwart von $CO_2$ gearbeitet werden kann, sofern die Gegenwart gewisser Oxadiazintrion-Anteile im Harz für die entsprechende Anwendung nicht stört. Führt man den Versuch in ansonsten analoger Weise mit quaternärem Ammoniumhydroxid, z.B. Benzyltrimethylammoniumhydroxid in alkoholischer Lösung, oder auch mit dem zum hier verwendeten Katalysator analogen quaternären Ammoniumfluorid, erhalten in der oben beschriebenen Weise mit dem Unterschied, daß der Schritt der HF Zugabe unterbleibt, durch, resultieren sofort nach Katalysatorzugabe trübe, z.T. Gelteilchen enthaltene Lösungen, die nicht zur Herstellung qualitativ hochwertiger, klarer HDI-Polyisocyanat-Harze geeignet sind.

**Beispiel 5** (erfindungsgemäß)

a) 2000 g HDI werden zunächst so vorbehandelt wie in Beispiel 2 beschrieben. Anschließend werden bei einer Innentemperatur von 50°C portionsweise insgesamt 12 ppm, bezogen auf Fluorid und die Masse an eingesetztem HDI, des Katalysators aus Beispiel 4 über einen Zeitraum von 90 min so zugetropft, daß die Innentemperatur 65°C nicht überschreitet. Bei einem NCO-Gehalt der Mischung von 43,0 % werden 0,3 g Dibutylphosphat zugegeben, eine weitere Stunde bei 50°C nachgerührt und anschließend aufgearbeitet wie unter Beispiel 4 beschrieben. Man erhält 520 g entsprechend 26 % Harzausbeute einer farblosen Trimerisatmischung die folgende Daten aufweist:

NCO-Gehalt: 23,6 %
Viskosität: 1050 mPa·s
Restmonomergehalt: 0,17 % HDI
Hazen-Farbzahl nach DIN 53 409: 56 APHA
molares Verhältnis von Iminooxadiazindionen zu Isocyanuraten: 0,8 : 1

Carbodiimide bzw. Uretonimine waren nicht nachweisbar.

b) Führt man den Versuch in prinzipiell analoger Weise bei einer Reaktionstemperatur von 150°C durch, einer Temperatur, die bei der Verwendung von Phosphin zur HDI-Oligomerisierung bereits deutlich zur Bildung von Carbodiimiden bzw. Uretoniminen führt, und stoppt nach Erreichen eines NCO-Gehaltes der Rohware von 39,0 % ab, erhält man in ca. 41 %iger Harzausbeute ein Harz mit einem NCO-Gehalt von 22,1 %, einem Restmonomergahlt von 0,1 % und einer Viskosität von 1900 mPa·s. Carbodiimide bzw. Uretonimine waren nicht nachweisbar.

**Beispiel 6** (erfindungsgemäß)

500 g des gemäß Beispiel 5 a) erhaltenen Produktes werden unter den gleichen Bedingungen, wie im Beispiel 3 aufgeführt destilliert und gereinigt. Man erhält 180 g einer zu mehr als 98 % aus reinen HDI-Trimeren, d.h. dem 1,3,5-Tris(6-isocyanatohexyl)isocyanurat und dem N,N',N"-Tris-(6-isocyanatohexyl)iminooxadiazindion), bestehenden Mischung mit im Vergleich zur Ausgangsoligomerenmischung unverändertem Verhältnis von Isocyanurat zu Iminooxadiazindion. Die Viskosität dieser Mischung beträgt 390 mPa·s bei 23°C, ihr NCO-Gehalt beläuft sich auf 25,0 %. Der Restmonomergehalt beträgt 0,1 % und verändert sich auch nach dreiwöchiger Lagerung bei 50°C im Trockenschrank nur unwesentlich (0,18 %). Carbodiimide bzw. Uretonimine waren nicht nachweisbar.

**Beispiel 7** (erfindungsgemäß)

Eine Mischung aus 84 g (0,5 mol) HDI und 111 g (0,5 mol) Isophorondiisocyanat (IPDI) wird in einem 250 ml Vierhalskolben mit Innenthermometer, Rührer, Rückflußkühler, Gaseinleitungsrohr sowie Dosiereinrichtung für die Katalysatorlösung zunächst bei Zimmertemperatur und einem Druck von ca. 0,1 mbar im Verlauf einer Stunde von im Diisocyanatgemisch gelösten Gasen befreit und anschließend unter Durchleiten eines schwachen Stickstoffstromes auf 60°C Innentemperatur erhitzt. Anschließend werden bei dieser Temperatur im Verlaufe von ca. 20 Minuten portionsweise insgesamt 1,614 g einer Lösung von 0,5 g Tetraethylammoniumfluorid-Hydrat (Fa. Aldrich) und 0,2 g Fluorwasserstoff in 5,6 g 2-Ethyl-1,3-hexandiol so zugesetzt, daß die Innentemperatur 70°C nicht überschreitet. Anschließend wird bei 60-70°C bis der NCO-Gehalt der Mischung bei 34,2 % liegt trimerisiert, durch Zugabe von 0,181 g Di-n-butylphosphat abgestoppt, eine weitere Stunde bei 60°C nachgerührt und anschließend von nicht umgesetzten monomeren Diisocyanaten durch Dünnschichtdestillation in einem Kurzwegverdampfer bei 0,1 mbar und einer Temperatur des Heizmediums von 170°C abgetrennt. Das so erhaltene, klare und nahezu farblose Harz (62,4 g entsprechend 32 % Ausbeute) weist in reiner Form eine Viskosität von 26 500 mPa·s, einen NCO-Gehalt von 18,8 % und Restmonomergehalte von 0,13 % an HDI und 0,27 % an IPDI auf. Das molare Verhältnis von Iminooxadiazindionen zu Isocyanuraten beträgt ca. 1:1. Carbodiimide bzw. Uretonimine waren nicht nachweisbar.

**Beispiel 8** (erfindungsgemäß)

100 g (0,51 mol) 1,3-Bis(isocyanatomethyl)cyclohexan (Fa. Aldrich) werden zunächst wie in Beispiel 2 beschrieben vorbehandelt und anschließend durch portionsweise Zugabe des in Beispiel 4 beschriebenen Katalysators (summarisch 42 ppm Fluorid) bei 58-60°C innerhalb von 3 Stunden auf einen NCO-Gehalt von 36,5 % trimerisiert. Anschließend wird durch Zugabe von 100 mg Di-n-octylphosphat abgestoppt, eine weitere Stunde bei 60°C nachgerührt und anschließend von nicht umgesetztem 1,3-Bis(isocyanatomethyl)cyclohexan durch Dünnschichtdestillation in einem Kurzwegverdampfer bei 0,2 mbar und einer Temperatur des Heizmediums von 140°C abgetrennt. Das so erhaltene, klare und nahezu farblose Harz (33,8 g entsprechend 33,8 % Ausbeute) weist einen NCO-Gehalt von 19,8 % auf und ist in reiner Form bei Zimmertemperatur (20-25°C) gerade noch fließfähig. Die Viskosität der 80 %igen Lösung in n-Butylacetat beträgt 1670 mPa·s bei einem NCO-Gehalt von 15,8 %. Der Restmonomergehalt beläuft sich auf 0,13 % an 1,3-Bis(isocyanatomethyl)cyclohexan. Die NMR-spektroskopische Analyse des Festharzes liefert folgendes Ergebnis: ca. 8 mol-% Uretdione, ca. 44 mol-% Isocyanurate sowie ca. 48 mol-% Iminooxadiazindione. Carbodiimide bzw. Uretonimine waren nicht nachweisbar.

Ein unter analogen Bedingungen erhaltenes, Iminooxadiazindiongruppen-freies Trimerisatharz, das im Unterschied zur erfindungsgemäßen Verfahrensweise unter Verwendung von 240 ppm, bezogen auf eingesetztes 1,3-Bis (isocyanatomethyl)cyclohexan und reines Benzyltrimethylammoniumhydroxid, einer Lösung von Benzyltrimethylammoniumhydroxid (Triton® B, Fa. Aldrich, geliefert als 40 %ige Lösung in Methanol und verdünnt auf 10 % Wirkstoff mit n-Butanol) hergestellt wurde, weist folgende Daten auf:

Harzausbeute: 34,0 %
NCO-Gehalt: 19,3 %
Viskosität der 80 %igen Lösung in n-Butylacetat: 2800 mPa·s
Restmonomergehalt: 0,2 % 1,3-Bis(isocyanatomethyl)cyclohexan.

Die NMR-spektroskopische Analyse des Festharzes liefert folgendes Ergebnis: ca. 4 mol-% Uretdione, ca. 1 mol-% Urethane, ca. 4 mol-% Allophanate und ca. 91 mol-% Isocyanurate.

## Patentansprüche

1. Verfahren zur Herstellung von Polyisocyanaten, **dadurch gekennzeichnet, daß** Isocyanate der Formel (I)

$$(OCN\text{-}CH_2)X \qquad (I),$$

in welcher

X für gegebenenfalls verzweigte, gegebenenfalls cyclische, gegebenenfalls weitere Heteroatome sowie Heteroatomgruppierungen (N, O, S) sowie mindestens eine weitere NCO-Gruppe enthaltenden $C_3$-$C_{20}$-Substituenten steht,

durch Polymerisation in Gegenwart von Hydrogen-Polyfluoriden der Formel (II)

$$M^{n+} n[F^- \cdot (HF)_m] \qquad (II),$$

in welcher
$m \geq 0,1$ ist

und

M für ein n-fach-geladenes Kation bzw. eine summarisch n-fach-geladene Kationenmischung steht,

als Oligomerisierungskatalysator hergestellt werden.

**2.** Verfahren nach Anspruch 1, **dadurch gekennzeichnet, daß** $m \geq 0,5$ ist.

**3.** Verfahren nach Anspruch 1, **dadurch gekennzeichnet, daß** $m \geq 1,0$ ist.

**4.** Verfahren gemäß Anspruch 1, **dadurch gekennzeichnet, daß** als Katalysator Tetraorganyl-ammonium- bzw. -phosphoniumhydrogenpolyfluoride der Formel (III)

$$R_4E^+ [F^- \cdot (BF)_m] \qquad (III),$$

wobei

E für N oder P steht,

R für gleiche oder verschiedene, gegebenenfalls verzweigte, gegebenenfalls cyclische, gegebenenfalls substituierte (O, N, Halogen) aliphatische, araliphatische sowie aromatische Reste mit jeweils 1 bis 25 C-Atomen steht und

m die unter Anspruch 1 definierte Bedeutung hat,

eingesetzt werden.

**5.** Verfahren gemäß Anspruch 4, **dadurch gekennzeichnet, daß** als Katalysator Tetraalkyl-ammoniumhydrogenpolyfluoride der Formel (III) eingesetzt werden wobei E für N steht, R für gleiche oder verschiedene, gegebenenfalls verzweigte, gegebenenfalls substituierte (O, N, Halogen) (cyclo)aliphatische sowie araliphatische Reste mit jeweils 1 bis 20 C-Atomen steht und m die unter Anspruch 1 definierte Bedeutung hat.

**6.** Verfahren gemäß Anspruch 5, **dadurch gekennzeichnet, daß** als Katalysator Benzyltrimethylammoniumhydrogenpolyfluoride der Formel (IV) eingesetzt werden:

$$C_6H_5CH_2(CH_3)_3N^+ [F^- \cdot (HF)_m] \qquad (IV),$$

wobei
m die unter Anspruch 1 definierte Bedeutung hat.

**7.** Verfahren gemäß Anspruch 5, **dadurch gekennzeichnet, daß** als Katalysator Tetraalkylammoniumhydrogenpolyfluoride der Formel (V) eingesetzt werden:

$$R_4N^+ [F^- \cdot (HF)_m] \qquad (V),$$

wobei

R für gleiche oder verschiedene, gegebenenfalls verzweigte, (cyclo)aliphatische Reste mit jeweils 1 bis 20 C-Atomen steht

und
m die unter Anspruch 1 definierte Bedeutung hat.

**8.** Verfahren gemäß Anspruch 7, **dadurch gekennzeichnet, daß** als Katalysator Tetraalkyl-ammoniumhydrogenpolyfluoride der Formel (VI) eingesetzt werden:

$$R^3(R')N^+ [F^- \cdot(HF)_m] \quad (VI),$$

wobei

R für gleiche oder verschiedene, gegebenenfalls verzweigte, aliphatische Reste mit jeweils 1 bis 15 C-Atomen steht,

R' für gegebenenfalls verzweigte, aliphatische Reste mit 1 bis 4 C-Atomen steht und

m die unter Anspruch 1 definierte Bedeutung hat.

**9.** Verfahren nach Anspruch 1, **dadurch gekennzeichnet, daß** die eingesetzten monomeren bzw. oligomeren Isocyanate Gemische oder reine Verbindungen von Spezies der allgemeinen Formel $(OCN\text{-}CH_2)X$ darstellen, wobei X die unter Anspruch 1 definierte Bedeutung hat, bis zur Umsetzung von 2-70 % der vor der Reaktion vorliegenden Isocyanatgruppen oligomerisiert wird, der Oligomerisierungskatalysator anschließend thermisch, adsorptiv oder durch Zugabe eines geeigneten Abstoppers desaktiviert wird und gegebenenfalls von noch vorhandenem Ausgangsisocyanat abgetrennt wird.

**Claims**

**1.** Process for the preparation of polyisocyanates, **characterized in that** isocyanates of the formula (I)

$$(OCN\text{-}CH_2)X \quad (I)$$

in which

X represents optionally branched, optionally cyclic $C_3$-$C_{20}$-substituents optionally containing further heteroatoms and heteroatom groupings (N, O, S) and at least one further NCO group,

are reacted by polymerization in the presence of hydrogen polyfluorides of the formula (II)

$$M^{n+}\ n\ [F^- \cdot(HF)_m] \quad (II)$$

in which
m is $\geq 0.1$
and

M represents a cation charged n-fold or a cation mixture charged n-fold in total,

as the oligomerization catalyst.

**2.** Process according to claim 1, **characterized in that** m is $\geq 0.5$.

**3.** Process according to claim 1, **characterized in that** m is $\geq 1.0$.

**4.** Process according to claim 1, **characterized in that** tetraorganyl-ammonium or -phosphonium hydrogen polyfluorides of the formula (III)

$$R_4E^+[F^- \cdot(HF)_m] \quad (III)$$

wherein

E represents N or P,

R represents identical or different, optionally branched, optionally cyclic, optionally substituted (O, N, halogen) aliphatic, araliphatic and aromatic radicals having in each case 1 to 25 C atoms and

m has the meaning defined under claim 1

are employed as the catalyst.

**5.** Process according to claim 4, **characterized in that** tetraalkylammonium hydrogen polyfluorides of the formula (III), wherein E represents N, R represents identical or different, optionally branched, optionally substituted (O, N, halogen) (cyclo)aliphatic and araliphatic radicals having in each case 1 to 20 C atoms and m has the meaning defined under claim 1, are employed as the catalyst.

**6.** Process according to claim 5, **characterized in that** benzyltrimethylammonium hydrogen polyfluorides of the formula (IV):

$$C_6H_5CH_2(CH_3)_3N^+[F^- \cdot (HF)_m] \quad (IV)$$

wherein

m has the meaning defined under claim 1

are employed as the catalyst.

**7.** Process according to claim 5, **characterized in that** tetraalkylammonium hydrogen polyfluorides of the formula (V):

$$R_4N^+[F^- \cdot (HF)_m] \quad (V)$$

wherein

R represents identical or different, optionally branched, (cyclo)aliphatic radicals having in each case 1 to 20 C atoms and

and
m has the meaning defined under claim 1
are employed as the catalyst.

**8.** Process according to claim 7, **characterized in that** tetraalkylammonium hydrogen polyfluorides of the formula (VI):

$$R_3(R')N^+[F^- \cdot (HF)_m] \quad (VI)$$

wherein

R represents identical or different, optionally branched, aliphatic radicals having in each case 1 to 15 C atoms,

R' represents optionally branched, aliphatic radicals having 1 to 4 C atoms and

m has the meaning defined under claim 1,

are employed as the catalyst.

9. Process according to claim 1, **characterized in that** the monomeric or oligomeric isocyanates employed are mixture or pure compounds of species of the general formula $(OCN-CH_2)X$, wherein X has the meaning defined under claim 1, oligomerization is carried out to conversion of 2-70% of the isocyanate groups present before the reaction, the oligomerization catalyst is then deactivated thermally, adsorptively or by addition of a suitable stopping agent, and starting isocyanate still present is optionally separated off.

## Revendications

1. Procédé de préparation de polyisocyanates, **caractérisé en ce que** l'on prépare des isocyanates de la formule (I) :

$$(OCN-CH_2)X \qquad (I)$$

dans laquelle
X représente des substituants en $C_3$-$C_{20}$ le cas échéant ramifiés, le cas échéant cycliques, contenant le cas échéant d'autres hétéroatomes ainsi que des groupements d'hétéroatomes (N, O, S), ainsi qu'au moins un autre radical NCO,
par polymérisation en présence d'hydrogénopolyfluorures de la formule (II) :

$$M^{n+}[F^{-}\cdot(HF)_m] \qquad (II)$$

dans laquelle
m est $\geq$ 0,1, et
M représente un cation chargé n-fois et respectivement, un mélange de cations chargé au total, n-fois, comme catalyseur d'oligomérisation.

2. Procédé suivant la revendication 1, **caractérisé en ce que** m est $\geq$ 0,5.

3. Procédé suivant la revendication 1, **caractérisé en ce que** m est $\geq$ 1,0.

4. Procédé suivant la revendication 1, **caractérisé en ce que** l'on met en oeuvre comme catalyseur, des hydrogénopolyfluorures de tétraorganylammonium ou -phosphonium de formule (III) :

$$R_4E^{+}[F^{-}\cdot(HF)_m] \qquad (III)$$

dans laquelle
E représente N ou P,
R représente des restes aliphatiques, araliphatiques ainsi que aromatiques, identiques ou différents, le cas échéant ramifiés, le cas échéant cycliques, le cas échéant substitués (O, N, halogène) , ayant chaque fois 1 à 25 atomes C, et
m a la signification définie à la revendication 1.

5. Procédé suivant la revendication 4, **caractérisé en ce que** l'on met en oeuvre comme catalyseur, des hydrogénopolyfluorures de tétralkylammonium de formule (III), où E représente N, R représente des restes (cyclo)aliphatiques ou araliphatiques, identiques ou différents, le cas échéant ramifiés, le cas échéant substitués (O, N, halogène), ayant chaque fois 1 à 20 atomes C, et m a la signification définie à la revendication 1.

6. Procédé suivant la revendication 5, **caractérisé en ce que** l'on met en oeuvre comme catalyseur, des hydrogénopolyfluorures de benzyltriméthyl-ammonium de formule (IV) :

$$C_6H_5CH_2(CH_3)_3N^{+}[F^{-}\cdot(HF)_m] \qquad (III)$$

dans laquelle

m a la signification définie à la revendication 1.

**7.** Procédé suivant la revendication 5, **caractérisé en ce que** l'on met en oeuvre comme catalyseur, des hydrogénopolyfluorures de tétralkylammonium de formule (V) :

$$R_4N+[F^- \cdot (HF)_m] \qquad (V)$$

dans laquelle
R représente des restes (cycle)aliphatiques, identiques ou différents, le cas échéant ramifiés, ayant chaque fois 1 à 20 atomes C, et
m a la signification définie à la revendication 1.

**8.** Procédé suivant la revendication 7, **caractérisé en ce que** l'on met en oeuvre comme catalyseur, des hydrogénopolyfluorures de tétralkylammonium de formule (VI) :

$$R_3(R')N^+[F^- \cdot (HF)_m] \qquad (VI)$$

dans laquelle
R représente des restes aliphatiques, identiques ou différents, le cas échéant ramifiés, ayant chaque fois 1 à 15 atomes C,
R' représente un reste aliphatique le cas échéant ramifié, ayant 1 à 4 atomes C, et
m a la signification définie à la revendication 1.

**9.** Procédé suivant la revendication 1, **caractérisé en ce que** les isocyanates monomères ou oligomères mis en oeuvre sont des mélanges ou des composés purs d'espèces de la formule générale $(OCN-CH_2)X$, où X a la signification définie à la revendication 1, jusqu'à une réaction de 2-70% par oligomérisation des radicaux isocyanate présents avant la réaction, le catalyseur d'oligomérisation est ensuite désactivé de manière thermique, par adsorption ou par addition d'un agent d'arrêt approprié, et le cas échéant, séparé de l'isocyanate de départ encore présent.